(19) Europäisches Patentamt
European Patent Office
Office européen des brevets

(11) Veröffentlichungsnummer: **0 087 000**
**B1**

(12) # EUROPÄISCHE PATENTSCHRIFT

(45) Veröffentlichungstag der Patentschrift: **13.11.85**

(51) Int. Cl.⁴: **C 07 D 307/86,** C 07 C 149/36, C 07 C 125/067, A 01 N 47/22

(21) Anmeldenummer: **83100864.4**

(22) Anmeldetag: **31.01.83**

(54) **N-Oxalylderivate von N-Methylcarbamaten, Verfahren zu ihrer Herstellung und ihre Verwendung als Schädlingsbekämpfungsmittel.**

(30) Priorität: **13.02.82 DE 3205195**

(43) Veröffentlichungstag der Anmeldung: **31.08.83 Patentblatt 83/35**

(45) Bekanntmachung des Hinweises auf die Patenterteilung: **13.11.85 Patentblatt 85/46**

(84) Benannte Vertragsstaaten: **AT BE CH DE FR GB IT LI NL SE**

(56) Entgegenhaltungen:
**EP - A - 0 022 498**
**US - A - 3 393 224**

**HOUBEN WEYL "Methoden der organischen Chemie", Band 8, 4. Auflage, 1952, GEORG THIEME VERLAG, Stuttgart**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Heywang, Gerhard, Dr., Nittumer Weg 4, D-5060 Bergisch-Gladbach 2 (DE)**
Erfinder: **Kühle, Engelbert, Dr., von-Bodelschwingh-Strasse 42, D-5060 Bergisch-Gladbach 2 (DE)**
Erfinder: **Behrenz, Wolfgang, Dr., Untergründemich 14, D-5063 Overath (DE)**
Erfinder: **Hammann, Ingeborg, Dr., Lutherstrasse 22, D-4330 Mülheim/Ruhr (DE)**
Erfinder: **Homeyer, Bernhard, Dr., Obere Strasse 28, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 087 000 B1

BUNDESDRUCKEREI BERLIN

**Beschreibung**

Die vorliegende Erfindung betrifft N-Oxalylderivate von Methylcarbamaten, Verfahren zu ihrer Herstellung und ihre Verwendung als Schädlingsbekämpfungsmittel.

Es ist bereits bekannt geworden, daß N-carboxylierte N-Methyl-carbamidsäurearylester (vergl. DE-A 2 132 936) und N-Chlorcarbonyl-N-methylcarbamidsäurearylester (vergl. DE-A 2 142 496) insektizide Eigenschaften haben. Ihre Wirkung ist jedoch, vor allem bei niedrigen Aufwandmengen nicht immer voll befriedigend.

Weiterhin sind aus EP-A 22 498 mit den erfindungsgemäßen Verbindungen strukturell verwandte Wirkstoffe bekannt, die an Stelle der Oxalylgruppe eine Carbonylfunktion im Molekül besitzen.

Es wurden nun N-Oxalyl-N-methyl-carbamidsäurearylester der Formel I gefunden

$$X-CO-CO-\overset{CH_3}{\overset{|}{N}}-CO-O-R^1 \qquad (I)$$

in welcher

$R^1$ für Phenyl, 2-Isopropylphenyl, 3-Isopropylphenyl, 2-Isopropoxyphenyl, 3,5-Dimethyl-4-methylmercaptophenyl, 3-Methyl-4-dimethylamino-phenyl, 4-Nitrophenyl, 2-Allyloxyphenyl, 3-sek.-Butyl-4-methylphenyl, 4-Methyl-3-isopropylphenyl, 2-Dimethylaminophenyl, 2-(1'-3'-Dioxolanyl-(2'))-phenyl, 2-(4',5'-Dimethyl-1',3'-dioxolanyl-(2'))-phenyl, 1-Naphthyl, 4-(1,1-Dimethylindanyl), 2,2-Dimethylbenzodioxolanyl, 2,2-Dimethyl-2,3-dihydrobenzofuranyl-(7) steht und

X für Methoxy, Ethoxy, Propoxy, iso-Propoxy, Butoxy, iso-Butoxy, sek.-Butoxy, tert.-Butoxy, Pentoxy, iso-Pentoxy, Hexoxy, iso-Hexoxy, Cyclopentoxy, Cyclohexoxy, Allyloxy, 2-Butenyloxy, 3-Butenyloxy, Propargyloxy, 2-Butinyloxy, 3-Butinyloxy, 2-Chlorethoxy, 2,2,2-Trichlorethoxy, 2-Fluorethoxy, 2,2,2-Trifluorethoxy, 2-Cyanoethoxy, 2-Nitroethoxy, 2-Methoxyethoxy, 2-Dimethylaminoethoxy, Phenoxy, 4-Chlor-phenoxy, 4-Methylphenoxy, 4-Methoxyphenoxy, 4-Dimethylaminophenoxy, 1-Naphtoxy, 2-Naphtoxy oder für Methylthio, Ethylthio, Butylthio, Phenylthio, 4-Chlorphenylthio, 4-Methylphenylthio oder für Amino, Methylamino, Dimethylamino, Ethylamino, Diethylamino, Propylamino, Isopropylamino, Dipropylamino, Diisopropylamino, Butylamino, iso-Butylamino, Dibutylamino, Diiso-Butylamino, Pyrrolidino, Piperidino, Morpholino, Thiomorpholino, N-Methylcyclohexyl amino, N-Phenylamino, N-Methyl-N-phenylamino, Diphenylamino, 4-Methyl-phenylamino, N-Methyl-N-4-methyl-phenylamino, N-Methyl-N-4-methoxyphenylamino, N-Methyl-N-4-chlorphenylamino steht.

Die neuen N-Oxalyl-N-methylcarbamidsäurearylester der Formel I

$$X-CO-CO-\overset{CH_3}{\overset{|}{N}}CO-O-R^1 \qquad (I)$$

in welcher X und $R^1$ die oben angegebene Bedeutung besitzen, erhält man, wenn man N-Chloroxalyl-N-methylcarbamidsäurearylester der Formel II

$$R^1-O-CO-\overset{CH_3}{\overset{|}{N}}-CO-CO-Cl \qquad (II)$$

in welcher

$R^1$ die oben angegebene Bedeutung hat,

mit Nukleophilen der Formel III

$$HX \qquad (III)$$

in welcher

X die oben angegebene Bedeutung hat,

gegebenenfalls in Gegenwart eines Verdünnungsmittels und/oder gegebenenfalls in Gegenwart einer Hilfsbase umsetzt.

Die N-Chloroxalyl-N-methylcarbamidsäurearylester der Formel II

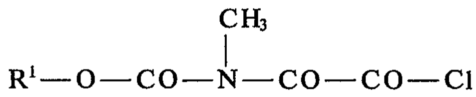

(II)

in welcher

$R^1$ die oben angegebene Bedeutung hat,

sind neu.

Man erhält sie indem man N-Methylcarbamidsäurearylester der Formel IV

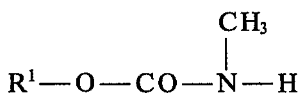

(IV)

in welcher

$R^1$ die oben angegebene Bedeutung hat,

mit Oxalylchlorid gegebenenfalls in Gegenwart eines Verdünnungsmittels und/oder gegebenenfalls in Gegenwart einer Hilfsbase umsetzt.

Die erfindungsgemäßen Verbindungen zeichnen sich durch gute Wirksamkeit als Insektizide, Akarizide und Nematozide aus.

Es ist ausgesprochen überraschend, daß sie bei günstiger Warmblütertoxizität eine höhere Wirkung zeigen als die aus dem Stand der Technik bekannten N-carboxylierten N-Methylcarbamate.

Die erfindungsgemäßen Verbindungen sind durch die obenstehende Formel I allgemein definiert.

Insbesondere sind bevorzugt Verbindungen der Formel I genannt, in welcher

$R^1$ für 3-iso-Propylphenyl, 2-iso-Propoxyphenyl, 3,5-Dimethyl-4-methylthiophenyl, 3-Methyl-4-dimethylaminophenyl, 2-(1',3'-Dioxolanyl-(2'))-phenyl-, 1-Naphthyl, 4-(1,1-Dimethylindanyl)-4-(2,2-Dimethylbenzodioxolanyl)-7-(2,2-Dimethyl-2,3-dihydrobenzofuranyl) und

X für Methoxy, Ethoxy, 2,2,2-Trichlorethoxy, 2,2,2-Trifluorethoxy, Butoxy, Allyloxy, Propargyloxy, Phenoxy, Methylthio, Phenylthio, Dimethylamino, Dibutylamino, Piperidino, Morpholino

steht.

Verwendet man 2,3-Dihydro-2,2-dimethyl-7-benzofuranyl-N-chloroxalyl-N-methylcarbamat (Formel V) und Methanol (Formel VI) als Ausgangsstoffe, so läßt sich der Reaktionsverlauf durch das folgende Formelschema wiedergeben:

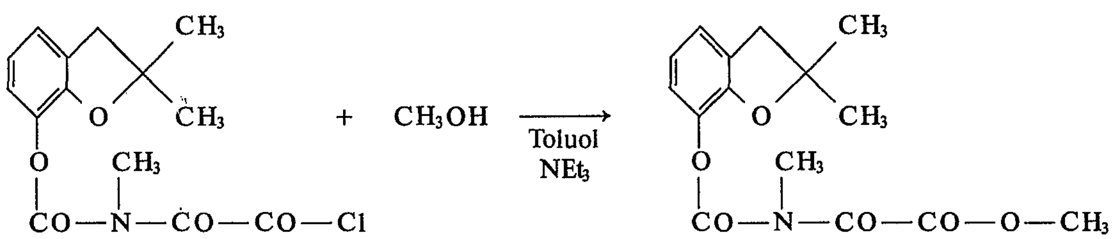


(V) (VI)

Die Ausgangsverbindungen der Formel II sind neu.

Bevorzugte Ausgangsverbindungen für die Verbindungen der Formel II sind N-Methylcarbamidsäurearylester der Formel IV, in denen der Rest $R^1$ die bei den Verbindungen der Formel I angegebenen bevorzugte Bedeutung besitzt.

Als Verdünnungsmittel für die Herstellung der erfindungsgemäßen Verbindungen eignen sich inerte organische Lösungsmittel. Hierzu gehören Ether wie Diethylether, Dioxan, Tetrahydrofuran, Kohlenwasserstoffe wie Benzol oder Toluol, chlorierte Kohlenwasserstoffe wie Methylenchlorid, Chloroform oder Chlorbenzole, weiterhin Nitrile, Ketone, Ester sowie Gemische aus diesen Lösungsmitteln.

Als säurebindende Mittel setzt man dem Reaktionsgemisch Natriumcarbonat, Natriumhydrogencarbonat oder eine tertiäre organische Base wie z. B. Triethylamin oder Benzyldimethylamin zu. Ist X—H ein Amin, so kann auch dieses als Base eingesetzt werden.

Die Reaktionstemperatur kann in einem größeren Bereich variiert werden. Im allgemeinen arbeitet man zwischen 0 und 100°C.

Gewöhnlich werden die Reaktionspartner in äquimolaren Mengen eingesetzt, doch auch die Verwendung einer Komponente im Überschuß ist möglich, bringt jedoch keine wesentlichen Vorteile mit sich.

Die neuen Verbindungen der Formel II werden erhalten, indem man N-Methylcarbamidsäurearylester der Formel IV mit Oxalylchlorid umsetzt.

Verwendet man 2,3-Dihydro-2,2-dimethyl-7-benzofuranyl-N-methylcarbamidsäureester (Formel VII) als Ausgangsstoff, so läßt sich der Reaktionsverlauf durch das folgende Formelschema wiedergeben.

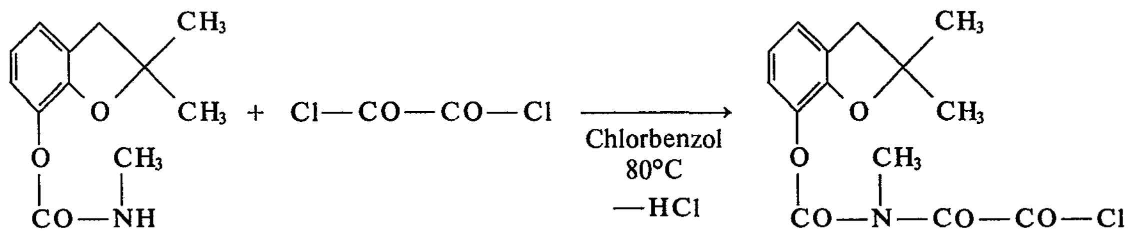


Als Verdünnungsmittel für diese Reaktion eignen sich inerte organische Lösungsmittel. Hierzu gehören Ether wie Diethylether, Dioxan, Tetrahydrofuran, Kohlenwasserstoffe wie Benzol, Toluol oder Xylol, chlorierte Kohlenwasserstoffe wie Chloroform, Dichlorethan oder Chlorbenzole, weiterhin Nitrile, Ketone, Ester sowie Gemische aus diesen Lösungsmitteln.

Der bei der Reaktion gebildete Chlorwasserstoff kann mit Gasen wie Luft, Stickstoff ausgeblasen werden oder er entweicht durch die Reaktionstemperatur oder man setzt als säurebindendes Mittel dem Reaktionsgemisch Natriumcarbonat, Natriumhydrogencarbonat oder eine tertiäre organische Base wie Triethylamin, Benzyldimethylamin oder N,N-Dimethylanilin zu.

Die Reaktionstemperatur kann in einem größeren Bereich variiert werden. Im allgemeinen arbeitet man zwischen 0 und 150°C, vorzugsweise zwischen 60 und 130°C, wenn keine säurebindenden Mittel zugefügt werden.

Gewöhnlich werden die Reaktionspartner in äquimolaren Mengen eingesetzt, doch auch die Verwendung einer Komponente im Überschuß ist möglich, bringt jedoch keine Vorteile mit sich.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Demaptera z. B. Forficula auricularia.
Aus der Ordnung der Isoptera z. B. Reticulitermes spp.
Aus der Ordnung der Anoplura z. B. Phylloxera vastatrix, Pemphigus spp., Pediculus humanus corporis, Haematopinus spp., Linognathus spp.
Aus der Ordnung der Mallophaga z. B. Trichodectes spp., Damalinea spp.
Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera, z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera, z. B. Diprion spp., Hoplocampa spp., Lasius spp., Monomorium pharaonis, Vespa spp.

Aus der Ordnung der Diptera, z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera, z. B. Xenopsylla cheopis, Ceratophyllus spp.

Aus der Ordnung der Arachnida, z. B. Scorpio maurus, Latrodectus mactans.

Aus der Ordnung der Acarina, z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; Als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gewichtsprozent Wirkstoff, vorzugsweise zwischen 0,0001 und 1 Gewichtsprozent liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Herstellungsbeispiele

### I. Herstellung der Ausgangsverbindungen

Ia.) 2,3-Dihydro-2,2-dimethyl-benzofuranyl-7-N-chloroxalyl-N-methyl-carbamat

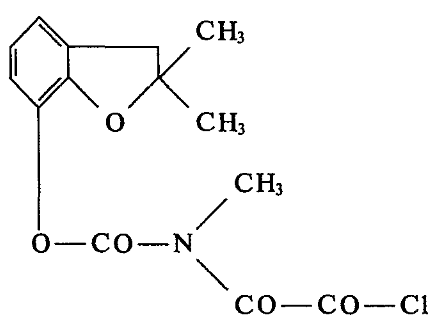

Zu 12,2 g 2,3-Dihydro-2,2-dimethyl-benzofuranyl-7-N-methylcarbamat in 100 ml Chlorbenzol werden 5,4 ml Oxalylchlorid zugetropft, und anschließend wird das Gemisch langsam auf 60—80°C erwärmt bis zum Ende der Gasentwicklung (etwa 4 Stunden). Die so entstandene Lösung kann für die Umsetzung zu den erfindungsgemäßen Verbindungen benutzt werden. Nach dem Abdestillieren des Chlorbenzols im Vakuum bleiben 15 g zähflüssiges Öl zurück, das nach dem Gaschromatogramm zu 96% aus der gewünschten Verbindung und zu 4% aus Chlorbenzol besteht. Die Verbindung siedet unzersetzt bei 161°C/0,01 mbar.

Ib.) Phenyl-N-chloroxalyl-N-methylcarbamat

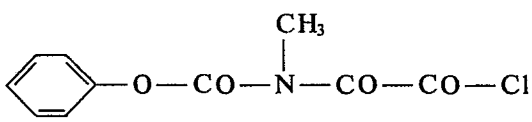

Analog der vorstehenden Vorschrift werden 151 g Phenyl-N-methyl-carbamat mit 127 g Oxalylchlorid in 1 l Chlorbenzol umgesetzt. 220 g (91%) farbloses Öl mit Siedepunkt 142°C/0,01 mbar.

Ic.) 3,5-Dimethyl-4-methylthio-phenyl-N-chloroxalyl-N-methyl-carbamat

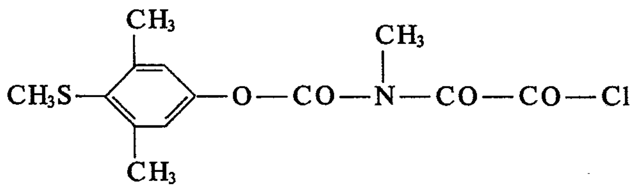

Analog zum Beispiel 1 werden 112,5 g 3,5-Dimethyl-4-methylthio-phenyl-N-methylcarbamat in 500 ml Chlorbenzol mit 63,5 g Oxalylchlorid umgesetzt. Nach Verdampfen des Lösungsmittels erhält man ein kristallines Produkt, das mit Diisopropylether gewaschen wird: 152 g (96%), Schmelzpunkt 100—104°C.

Analog erhält man

Id.)

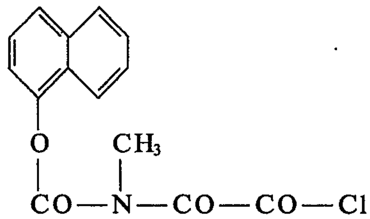

Ausbeute 100%
Fp. 85–90°C

Ie.)

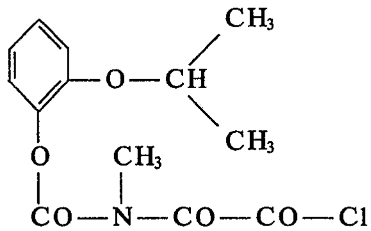

Ausbeute 97%
Fp. 55–65°C

If.) 3-Methyl-4-dimethylamino-phenyl-N-chloroxalyl-N-methylcarbamat-hydrochlorid

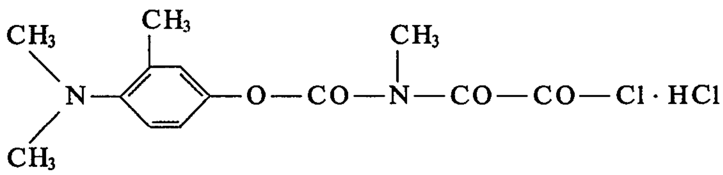

Analog zur Vorschrift vom Beispiel 1 werden 104 g 3-Methyl-4-dimethylaminophenyl-N-methylcarbamat in 500 ml Chlorbenzol mit 63,5 g Oxalylchlorid versetzt. Das Reaktionsgemisch wird 4 Stunden auf 60°C erhitzt. Dabei scheidet sich eine ölige Schicht an der Wand des Reaktionsgefäßes ab. Man dekantiert das Chlorbenzol und digeriert den Rückstand mit Diisopropylether.

60 g (40%) sehr hygroskopisches farbloses Pulver mit Schmelzbereich 52–69°C.

## II. Herstellung der Verbindungen der Formel I

1. 2-[N-7-(2,3-Dihydro-2,2-dimethylbenzofuranyl)-oxycarbonyl-N-methyl]-amino-2-oxo-ethansäuremethylester

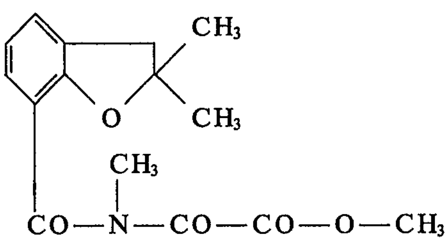

Zu 8,7 g 2,3-Dihydro-2,2-dimethylbenzofuranyl-7-N-oxalyl-N-methylcarbamat in 20 ml Chlorbenzol werden bei Raumtemperatur tropfenweise zuerst 1 g Methanol und dann 2,8 g Triethylamin zugegeben. Man rührt 30 Min. nach, filtriert, wäscht die organische Phase zweimal mit 10 ml Wasser. Nach dem Trocknen über Natriumsulfat wird das Lösungsmittel im Vakuum abdestilliert, der Rückstand ist ein leicht bräunliches Öl. 8 g (93%), $n_D^{20}$: 1,5324.

Auf entsprechende Weise wurden die folgenden Oxalsäurederivate von Carbamaten hergestellt:

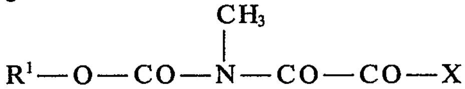

| $R^1$ | X | Ausbeute (%) | $n_D^{20}$ | Fp. (°C) |
|---|---|---|---|---|
| 2. 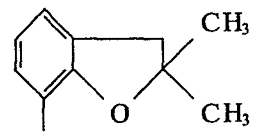 | $-O-C_2H_5$ | 100 | 1,5208 | |
| 3. desgl. | $-O-C_4H_9$ | 92 | 1,5150 | |
| 4. desgl. | $-O-CH_2-CF_3$ | 68 | 1,483 | |
| 5. desgl. | $-O-CH_2-CCl_3$ | 60 | | 75-78 |
| 6. desgl. | $-O-CH_2-CH=CH_2$ | 100 | 1,5242 | |
| 7. desgl. | $-O-CH_2-C\equiv CH$ | 64 | 1,5281 | |
| 8. desgl. | $-O-C_6H_5$ | 58 | | 118-125 |
| 9. desgl. | $-S-C_6H_5$ | 90 | | 78-81 |
| 10. desgl. | $-N(C_4H_9)_2$ | 97 | 1,5171 | |
| 11. desgl. | 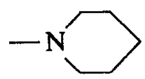 | 84 | | 111-118 |
| 11a. desgl. | 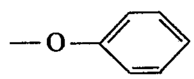 | 72 | | 122 |
| 12. 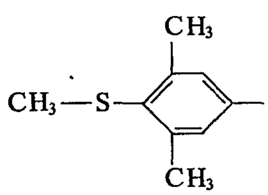 | $-O-CH_3$ | 96 | | 66 |
| 13. desgl. | $-O-C_4H_9$ | 85 | | 68-74 |
| 14. 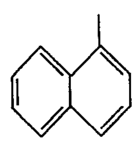 | $-O-C_4H_9$ | 86 | 1,5573 | |
| 15. 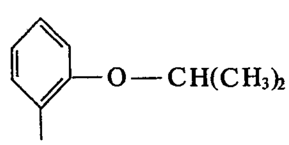 | $-O-CH_3$ | 86 | 1,5072 | |
| 16. desgl. | $-O-C_4H_9$ | 83 | 1,4992 | |

## Beispiel A

### Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z. B. die folgenden Verbindungen der Herstellungsbeispiele überlegene Wirksamkeit gegenüber dem Stand der Technik: 1, 2, 3, 4, 5, 6, 7, 9, 11, 11a.

Beispiel B

Doralis-Test (systemische Wirkung)

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z. B. die folgenden Verbindungen der Herstellungsbeispiele überlegene Wirksamkeit gegenüber dem Stand der Technik: 1, 2, 3, 4, 5, 6, 7, 9, 11, 11a.

Beispiel C

Tetranychus-Test (resistent)

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 4.

Beispiel D

Grenzkonzentrations-Test/Wurzelsystemische Wirkung

| | | |
|---|---|---|
| Testinsekt: | Myzus persicae | |
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen

oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1, 2, 3, 4, 5, 6, 9, 11 und 11a.

Beispiel E

Grenzkonzentrations-Test/Wurzelsystemische Wirkung

| | | |
|---|---|---|
| Testinsekt: | Phaedon Cochleariae-Larven | |
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1, 2, 3, 4, 6, 7, 9, 11, 11a.

Beispiel F

Grenzkonzentrations-Test/Bodeninsekten

| | | |
|---|---|---|
| Testinsekt: | Phorbia Antiqua-Maden (im Boden) | |
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1, 2, 3, 4, 5, 6, 9, 10, 11, 11a.

Beispiel G

Grenzkonzentrations-Test/Nematoden

| | | |
|---|---|---|
| Testnematode: | Meloidogyne incognita | |
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach 4 Wochen werden die Salatwurzeln auf Nematodenbefall (Wurzelgallen) untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigen z. B. die folgenden Verbindungen der Herstellungsbeispiele überlegene Wirksamkeit gegenüber dem Stand der Technik: 10.

Beispiel H

$LT_{100}$-Test für Dipteren

Testtiere: Musca domestica (resistent)
Zahl der Testtiere: 25
Lösungsmittel: Aceton

2 Gewichtsteile Wirkstoff werden in 1000 Volumteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100%igen knock-down-Effekt notwendig ist.

Bei diesem Test zeigen z. B. die folgenden Verbindungen der Herstellungsbeispiele überlegene Wirkung gegenüber dem Stand der Technik: 4, 5, 9, 11.

**Patentansprüche**

1. N-Oxalyl-N-methyl-carbamidsäurearylester der Formel I

$$X-CO-CO-\underset{}{N}(CH_3)-CO-O-R^1 \qquad (I)$$

in welcher

$R^1$ für Phenyl, 2-Isopropylphenyl, 3-Isopropylphenyl, 2-Isopropoxyphenyl, 3,5-Dimethyl-4-methylmercaptophenyl, 3-Methyl-4-dimethylamino-phenyl, 4-Nitrophenyl, 2-Allyloxyphenyl, 3-sek.-Butyl-4-methylphenyl, 4-Methyl-3-isopropylphenyl, 2-Dimethylaminophenyl, 2-(1'-3'-Dioxolanyl(2'))-phenyl, 2-(4',5'-Dimethyl-1',3'-dioxolanyl-(2'))-phenyl, 1-Naphthyl, 4-(1,1-Dimethylindanyl), 2,2-Dimethylbenzodioxolanyl, 2,2-Dimethyl-2,3-dihydrobenzofuranyl-(7) steht und

X für Methoxy, Ethoxy, Propoxy, iso-Propoxy, Butoxy, iso-Butoxy, sek.-Butoxy, tert.-Butoxy, Pentoxy, iso-Pentoxy, Hexoxy, iso-Hexoxy, Cyclopentoxy, Cyclohexoxy, Allyloxy, 2-Butenyloxy, 3-Butenyloxy, Propargyloxy, 2-Butinyloxy, 3-Butinyloxy, 2-Chlorethoxy, 2,2,2-Trichlorethoxy, 2-Fluorethoxy, 2,2,2-Trifluorethoxy, 2-Cyanoethoxy, 2-Nitroethoxy, 2-Methoxyethoxy, 2-Dimethylaminoethoxy, Phenoxy, 4-Chlor-phenoxy, 4-Methylphenoxy, 4-Methoxyphenoxy, 4-Dimethylaminophenoxy, 1-Naphthoxy, 2-Naphthoxy oder für Methylthio, Ethylthio, Butylthio, Phenylthio, 4-Chlorphenylthio, 4-Methylphenylthio oder für Amino, Methylamino, Dimethylamino, Ethylamino, Diethylamino, Propylamino, Isopropylamino, Dipropylamino, Diisopropylamino, Butylamino, iso-Butylamino, Dibutylamino, Diiso-butylamino, Pyrrolidino, Piperidino, Morpholino, Thiomorpholino, N-Methylcyclohexylamino, N-Phenylamino, N-Methyl-N-phenylamino, Diphenylamino, 4-Methyl-phenylamino, N-Methyl-N-4-methyl-phenylamino, N-Methyl-N-4-methoxyphenylamino, N-Methyl-N-4-chlorphenylamino steht.

2. Verfahren zur Herstellung der N-Oxalyl-N-methylcarbamidsäurearylester der Formel I

$$X-CO-CO-N(CH_3)-CO-O-R^1 \qquad (I)$$

in welcher

X und
$R^1$ die im Anspruch 1 angegebene Bedeutung haben,

dadurch gekennzeichnet, daß man N-Chloroxalyl-N-methylcarbamidsäurearylester der Formel II

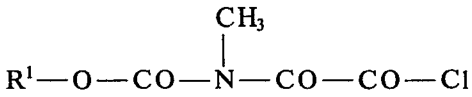

(II)

in welcher

$R^1$ die oben angegebene Bedeutung hat,

mit Nukleophilen der Formel III

HX (III)

in welcher

X die oben angegebene Bedeutung hat,

gegebenenfalls in Gegenwart eines Verdünnungsmittels und/oder gegebenenfalls in Gegenwart einer Hilfsbase umsetzt.

3. N-Chloroxalyl-N-methylcarbamidsäurearylester der Formel II

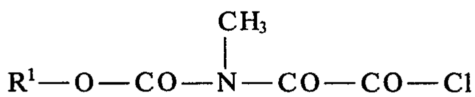

(II)

in welcher

$R^1$ die im Anspruch 1 angegebene Bedeutung hat.

4. Verfahren zur Herstellung der N-Chloroxalyl-N-methylcarbamidsäurearylester der Formel II gemäß Anspruch 3, dadurch gekennzeichnet, daß man N-Methylcarbamidsäurearylester der Formel IV

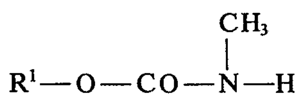

(IV)

in welcher

$R^1$ die im Anspruch 3 angegebene Bedeutung hat,

mit Oxalylchlorid gegebenenfalls in Gegenwart eines Verdünnungsmittels und/oder gegebenenfalls in Gegenwart einer Hilfsbase umsetzt.

5. Schädlingsbekämpfungsmittel, gekennzeichnet durch einen Gehalt an mindestens einem N-Oxalyl-N-methylcarbamidsäurearylester der Formel I.

6. Verwendung von N-Oxalyl-N-methyl-carbamidsäurearylester der Formel I zur Bekämpfung von Schädlingen.

7. Verfahren zur Bekämpfung von Schädlingen, dadurch gekennzeichnet, daß man N-Oxalyl-N-methyl-carbamidsäurearylester der Formel I auf Schädlinge und/oder ihren Lebensraum einwirken läßt.

8. Verfahren zur Herstellung von Schädlingsbekämpfungsmitteln, dadurch gekennzeichnet, daß man N-Oxalyl-N-methyl-carbamidsäurearylester der Formel I mit Streckmitteln und/oder oberflächenaktiven Mitteln vermischt.

**Claims**

1. N-Oxalyl-N-methyl-carbamic acid aryl esters of the formula I

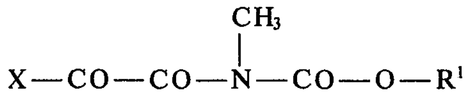

(I)

in which

$R^1$ represents phenyl, 2-isopropylphenyl, 3-isopropylphenyl, 2-isopropoxyphenyl, 3,5-dimethyl-4-methyl-mercaptophenyl, 3-methyl-4-dimethylamino-phenyl, 4-nitrophenyl, 2-allyloxyphenyl, 3-sec.-butyl-4-methyl-phenyl, 4-methyl-3-isopropylphenyl, 2-dimethylaminophenyl, 2-(1',3'-dioxolan-2'yl)-phenyl, 2-(4',5'-dimethyl-1',3'-dioxolan-2'-yl)-phenyl, naphth-1-yl, 4-(1,1-dimethylindanyl), 2,2-dimethylbenzodioxolanyl or 2,2-dimethyl-2,3-dihydrobenzo-furanyl-(7) and

X represents methoxy, ethoxy, propoxy, isopropoxy, butoxy, isobutoxy, sec.-butoxy, tert.-butoxy, pentoxy, isopentoxy, hexoxy, isohexoxy, cyclopentoxy, cyclohexoxy, allyloxy, but-2-enyloxy, but-3-enyloxy, propargyloxy, but-2-inyloxy, but-3-inyloxy, 2-chloroethoxy, 2,2,2-trichloroethoxy, 2-fluoroethoxy, 2,2,2-trifluoroethoxy, 2-cyanoethoxy, 2-nitroethoxy, 2-methoxyethoxy, 2-dimethylaminoethoxy, phenoxy, 4-chlorophenoxy, 4-methylphenoxy, 4-methoxyphenoxy, 4-dimethylaminophenoxy, 1-naphthoxy, 2-naphthoxy or methylthio, ethylthio, butylthio, phenylthio, 4-chlorophenylthio, 4-methylphenylthio or amino, methylamino, dimethylamino, ethylamino, diethylamino, propylamino, isopropylamino, dipropylamino, diisopropylamino, butylamino, isobutylamino, dibutylamino, diisobutylamino, pyrrolidino, piperidino, morpholino, thiomorpholino, N-methylcyclohexylamino, N-phenylamino, N-methyl-N-phenylamino, diphenylamino, 4-methylphenylamino, N-methyl-N-4-methylphenylamino, N-methyl-N-4-methoxyphenylamino or N-methyl-N-4-chlorophenylamino.

2. Process for the preparation of the N-oxalyl-N-methylcarbamic acid aryl esters of the formula I

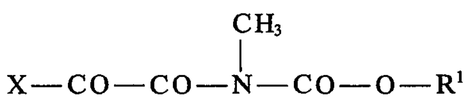

(I)

in which

X and
$R^1$ have the meaning given in Claim 1,

characterised in that N-chloroxalyl-N-methyl-carbamic acid aryl esters of the formula II

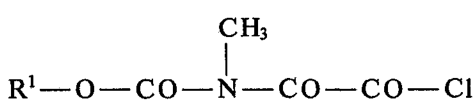

(II)

in which

$R^1$ has the abovementioned meaning,

are reacted with nucleophiles of the formula III

HX (III)

in which

X has the abovementioned meaning,

if appropriate in the presence of a diluent and/or if appropriate in the presence of an auxiliary base.

3. N-Chloroxalyl-N-methylcarbamic acid aryl esters of the formula II

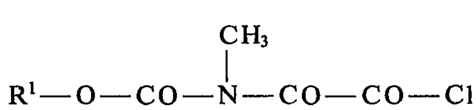

(II)

in which

$R^1$ has the meaning given in Claim 1.

4. Process for the preparation of the N-chloroxalyl-N-methyl-carbamic acid aryl esters of the formula II according to Claim 3, characterised in that N-methyl-carbamic acid aryl esters of the formula IV

$$R^1—O—CO—\overset{CH_3}{\overset{|}{N}}—H \qquad (IV)$$

in which

$R^1$ has the meaning given in Claim 3,

are reacted with oxalyl chloride, if appropriate in the presence of a diluent and/or if appropriate in the presence of an auxiliary base.

5. Agents for combating pests, characterised in that they contain at least one N-oxalyl-N-methyl-carbamic acid aryl ester of the formula I.

6. Use of N-oxalyl-N-methyl-carbamic acid aryl ester of the formula I for combating pests.

7. Method of combating pests, characterised in that N-oxalyl-N-methyl-carbamic acid aryl esters of the formula I are allowed to act on pests and/or their environment.

8. Process for the preparation of agents for combating pests, characterised in that N-oxalyl-N-methyl-carbamic acid aryl esters of the formula I are mixed with extenders and/or surface-active agents.

**Revendications**

1. Esters aryliques d'acide N-oxalyl-N-méthylcarbamique de formule I

$$X—CO—CO—\overset{CH_3}{\overset{|}{N}}—CO—O—R^1 \qquad (I)$$

dans laquelle

$R^1$ est un groupe phényle, 2-isopropylphényle, 3-isopropylphényle, 2-isopropoxyphényle, 3,5-diméthyl-4-méthylmercaptophényle, 3-méthyl-4-diméthylaminophényle, 4-nitrophényle, 2-allyloxyphényle, 3-sec.-butyl-4-méthylphényle, 4-méthyl-3-isopropylphényle, 2-diméthylaminophényle, 2-(1',3'-dioxolanyl(2'))-phényle, 2-(4',5'-diméthyl-1',3'-dioxolanyl-(2'))-phényle, 1-naphthyle, 4-(1,1-diméthylindanyle), 2,2-diméthylbenzodioxolanyle, 2,2-diméthyl-2,3-dihydrobenzofuranyl(7) et

X est un groupe méthoxy, éthoxy, propoxy, isopropoxy, butoxy, isobutoxy, sec.-butoxy, tertiobutoxy, pentoxy, isopentoxy, hexoxy, isohexoxy, cyclopentoxy, cyclohexoxy, allyloxy, 2-butényloxy, 3-butényloxy, propargyloxy, 2-butynyloxy, 3-butynyloxy, 2-chloréthoxy, 2,2,2-trichloréthoxy, 2-fluoréthoxy, 2,2,2-trifluoréthoxy, 2-cyanéthoxy, 2-nitroéthoxy, 2-méthoxyéthoxy, 2-diméthylaminoéthoxy, phénoxy, 4-chlorophénoxy, 4-méthylphénoxy, 4-méthoxyphénoxy, 4-diméthylaminophénoxy, 1-naphtoxy, 2-naphtoxy ou un groupe méthylthio, éthylthio, butylthio, phénylthio, 4-chlorophénylthio, 4-méthylphénylthio ou un groupe amino, méthylamino, diméthylamino, éthylamino, diéthylamino, propylamino, isopropylamino, dipropylamino, diisopropylamino, butylamino, isobutylamino, dibutylamino, diisobutylamino, pyrrolidino, pipéridino, morpholino, thiomorpholino, N-méthylcyclohexylamino, N-phénylamino, N-méthyl-N-phénylamino, diphénylamino, 4-méthylphénylamino, N-méthyl-N-4-méthylphénylamino, N-méthyl-N-4-méthoxyphénylamino, N-méthyl-N-4-chlorophénylamino.

2. Procédé de production des esters aryliques d'acide N-oxalyl-N-méthylcarbamide de formule I

$$X—CO—CO—\overset{CH_3}{\overset{|}{N}}—CO—O—R^1 \qquad (I)$$

dans laquelle

X et
$R^1$ ont la définition indiquée dans la revendication 1, caractérisé en ce qu'on fait réagir des esters aryliques d'acide N-chloroxalyl-N-méthylcarbamique de formule II

$$R^1—O—CO—\overset{CH_3}{\overset{|}{N}}—CO—CO—Cl \qquad (II)$$

dans laquelle

$R^1$ a la définition indiquée ci-dessus,

avec des nucléophiles de formule III

$$HX \qquad (III)$$

dans laquelle

X a la définition indiquée ci-dessus,

éventuellement en présence d'un diluant et/ou en la présence éventuelle d'une base auxiliaire.

3. Esters aryliques d'acide N-chloroxalyl-N-méthylcarbamique de formule II

$$R^1—O—CO—\overset{CH_3}{\overset{|}{N}}—CO—CO—Cl \qquad (II)$$

dans laquelle

$R^1$ a la définition indiquée ci-dessus.

4. Procédé de production des esters aryliques d'acide N-chloroxalyl-N-méthylcarbamique de formule II suivant la revendication 3, caractérisé en ce qu'on fait réagir des esters aryliques d'acide N-méthylcarbamique de formule IV

$$R^1—O—CO—\overset{CH_3}{\overset{|}{N}}—H \qquad (IV)$$

dans laquelle

$R^1$ a la définition indiquée dans la revendication 3,

avec du chlorure d'oxalyle, le cas échéant en présence d'un diluant et/ou en la présence éventuelle d'une base auxiliaire.

5. Compositions pesticides, caractérisées par une teneur en au moins un ester arylique d'acide N-oxalyl-N-méthylcarbamique de formule I.

6. Utilisation d'esters aryliques d'acide N-oxalyl-N-méthylcarbamique de formule I pour combattre des parasites.

7. Procédé pour combattre des parasites, caractérisé en ce qu'on fait réagir des esters aryliques d'acide N-oxalyl-N-méthylcarbamique de formule I sur des parasites et/ou sur leur habitat.

8. Procédé de préparation de compositions pesticides, caractérisé en ce qu'on mélange des esters aryliques d'acide N-oxalyl-N-méthylcarbamique de formule I avec des diluants et/ou des agents tensio-actifs.